# EUROPEAN PATENT APPLICATION

(11) **EP 1 564 719 A2**
(43) Date of publication of application: **17.08.2005**
(21) Application number: 04030852.0
(22) Date of filing: 28.12.2004
(51) Int. Cl.: G10K 9/20

(54) **Sound emission device and siren, particularly for alarm systems for motor vehicles, motorcycles, enclosed spaces and the like**

(30) Priority: 11.02.2004 IT MI20040223
(71) Applicant: S.I.S.A. S.R.L., 21040 Gerenzano (Varese) (IT)
(72) Inventor: Caranci, Gianfranco, 21047 Saronno (Varese) (IT)
(74) Representative: Forattini, Amelia

(57) **Abstract**

A sound emission device (1) for alarm systems for motor vehicles, motorcycles, enclosed spaces and the like, including a diffuser (2) having a membrane (4) accommodated therein, the membrane being made to vibrate by a transducer means (5). The membrane and the diffuser are formed monolithically.

## Description

The present invention relates to a sound emission device, particularly for alarm systems for motor vehicles, motorcycles, enclosed spaces and the like.

As is known, alarm systems for motor vehicles, motorcycles, enclosed spaces and the like, usually have a sound emission device that is generally constituted by a diffuser with a membrane that is made to vibrate or by an acoustic transducer of the piezoelectric or magnetodynamic type.

Currently, the body of the diffuser, which is generally cylindrical, has a partition that is provided with an opening that is completely occupied by the membrane. The coupling between the membrane and the partition of the diffuser is provided so as to ensure hermetic tightness of the resulting assembly.

However, often this hermetic tightness cannot be achieved absolutely and there may be passages, for example of water, from one side of the partition to the other through the edges where the membrane and the partition of the diffuser are coupled to each other.

Since the sound emission device comprises a printed circuit, a battery and the like, which are connected to the piezoelectric or magnetodynamic acoustic transducer, the penetration of water or of other foreign matter in the diffuser body, where these components are arranged, is absolutely disadvantageous in terms of optimum operation of the sound emission device.

Accordingly, all manufacturers of sound emission devices or sirens for anti-theft devices for cars and alarm systems for enclosed spaces in general would like to have a device that is absolutely hermetic in order to ensure optimum operation thereof.

Moreover, the presence of two separate components, i.e., the body of the diffuser and the membrane, increases assembly times and costs.

Also, the membrane may not be associated with the partition of the diffuser always in an absolutely identical manner for all the devices that are assembled, and therefore it is difficult to achieve optimization and repeatability of the frequency response of the membrane.

The aim of the present invention is to provide a sound emission device for alarm systems for motor vehicles, motorcycles, enclosed spaces and the like that is completely impermeable to external agents that may penetrate the body of the diffuser.

An object of the present invention is to provide a sound emission device that allows reduced assembly times and costs with respect to the prior art devices.

A further object of the present invention is to provide a sound emission device that allows to vary the passband according to the shape of the membrane.

A further object of the present invention is to provide a sound emission device that is highly reliable, relatively simple to provide and at competitive costs.

This aim and these and other objects that will become better apparent hereinafter are achieved by a sound emission device for alarm systems for motor vehicles, motorcycles, enclosed spaces and the like, which includes a diffuser inside which at least one membrane is accommodated, the membrane being suitable to be made to vibrate by a transducer means, characterized in that the membrane and the diffuser are formed monolithically.

Further characteristics and advantages of the invention will become better apparent from the following detailed description of preferred but not exclusive embodiments of the device according to the present invention, illustrated by way of non-limiting example in the accompanying drawings, wherein:
Figure 1 is a transverse sectional view of a first embodiment of the device according to the present invention;
Figure 2 is a perspective view of the diffuser and of the membrane of the sound emission device according to the present invention;

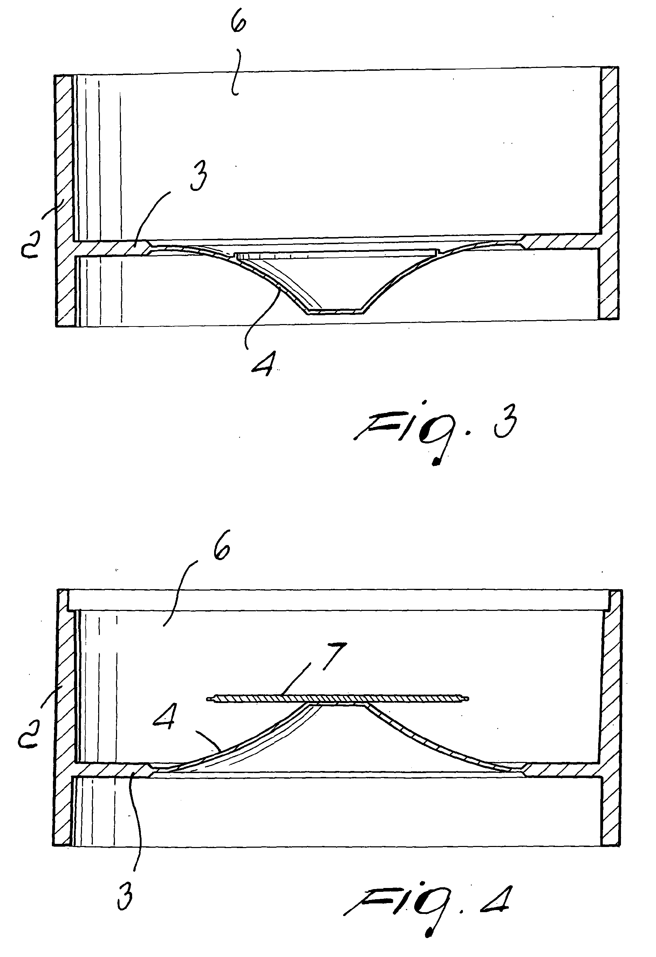

Figure 3 is a transverse sectional view of the sound emission device shown in Figure 1, with the transducer removed;
Figure 4 is a transverse sectional view of a second embodiment of the sound emission device according to the present invention;
Figure 5 is a perspective exploded view of a siren provided with the sound emission device according to the invention;
Figure 6 is a side section view of a siren provided with the sound emission device according to the invention;
Figure 7 is a side section view of a further embodiment of a siren provided with the sound emission device according to the invention.

With reference to the figures cited above, a sound emission device according to the invention, generally designated by the reference numeral 1, comprises a diffuser body 2 which has a substantially cylindrical shape and is hollow.

The diffuser body 2 is internally provided with at least one partition 3 that has an opening for accommodating a membrane 4.

According to the invention, the membrane 4 is formed in a single molding process, without discontinuities, together with the body 2 of the diffuser. Substantially, the membrane 4 is co-molded with the diffuser 2, thus ensuring a perfect seal of the coupling between the body 2 of the diffuser and the membrane 4.

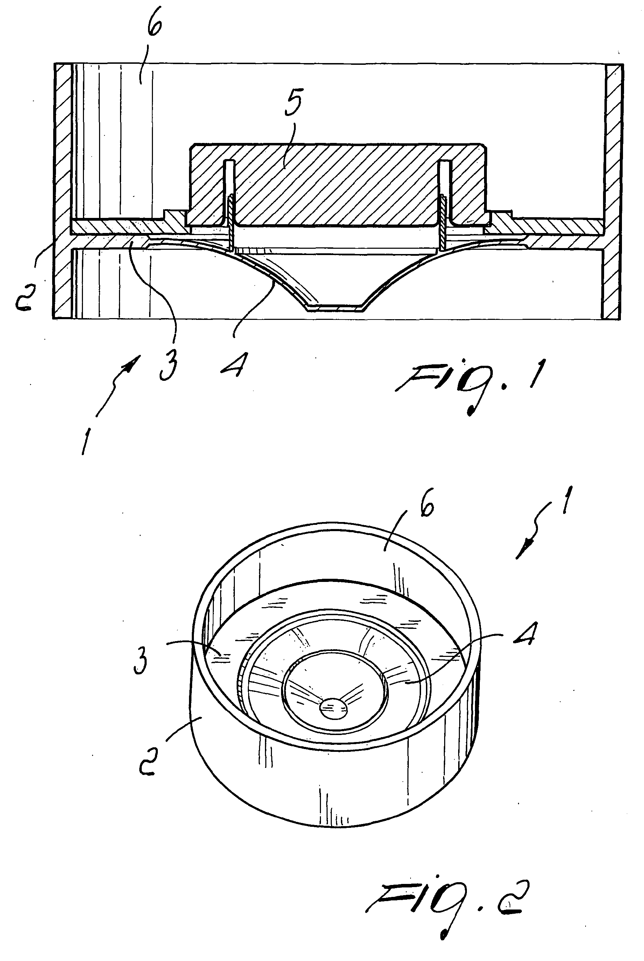

As visible in Figure 1, the membrane 4 can be shaped like a frustum or, not shown, like a portion of a sphere, with an exponential shape having a circular, square or rectangular cross-section.

In Figure 1, the membrane is shaped like a frustum in which the vertex of the cone is directed toward the outside of the diffuser body 2. In this case, therefore, the membrane 4 is actuated by a magnetodynamic transducer 5 which is connected to the partition 3 of the transducer, on the opposite side with respect to the side where the membrane 4 is arranged.

In a region, designated by the reference numeral 6, of the body 2 of the diffuser within which the magnetodynamic transducer 5 is arranged there are also a printed circuit, a battery, etc.

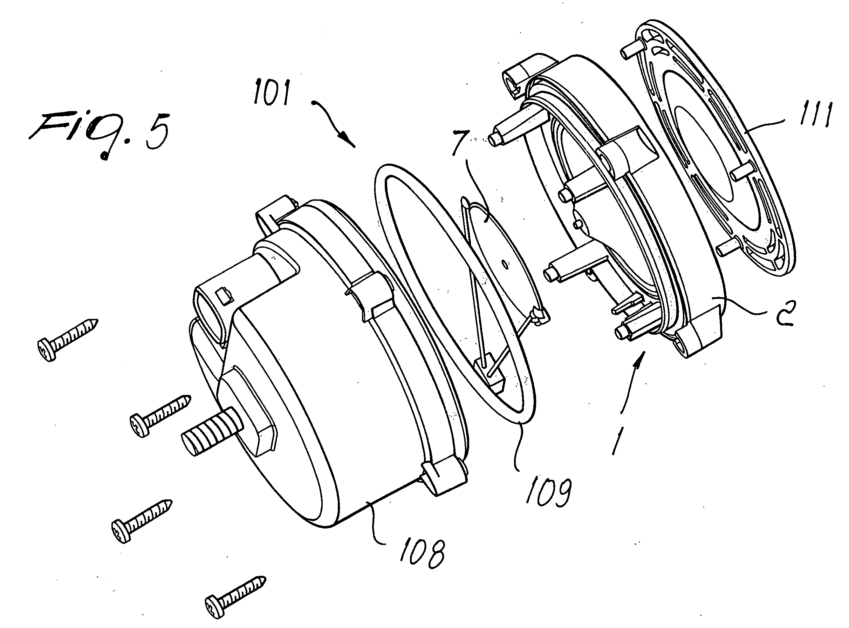

In the second embodiment of the device according to the invention, shown in Figure 4, the membrane 4 is again frustum-shaped and in this case is directed toward the inside of the diffuser body 2, i.e., is directed within the region 6.

In this case, a piezoelectric transducer 7 arranged adjacent to the membrane is used. In both embodiments, the membrane and the diffuser are co-molded in a single molding process.

By varying the dimensions, shapes, thicknesses and materials used, it is possible to obtain different frequency responses from the membrane, so as to optimize its use according to the application.

The body 2 of the diffuser and the membrane 4 can be made of the same material or of different materials.

The materials are preferably polymers for injection-molding.

Essentially, the co-molding of the membrane 4 together with the body 2 of the diffuser provides the advantage of having the front part of the sound emission device completely isolated from the internal volume that accommodates the acoustic transducer and the electronic components that constitute the siren or sound emission device.

Figure 5 illustrates a siren 101 comprising a sound emission device 1 according to the invention.

More particularly, the siren 101 comprises a casing 108 which is sealingly closed by the diffuser body 2 of the sound emission device 1 according to the invention.

A seal member, for example an O-ring 109, is provided between the casing 108 and the diffuser body 2 and a protective cover 111 is provided at the front of the diffuser body 2.

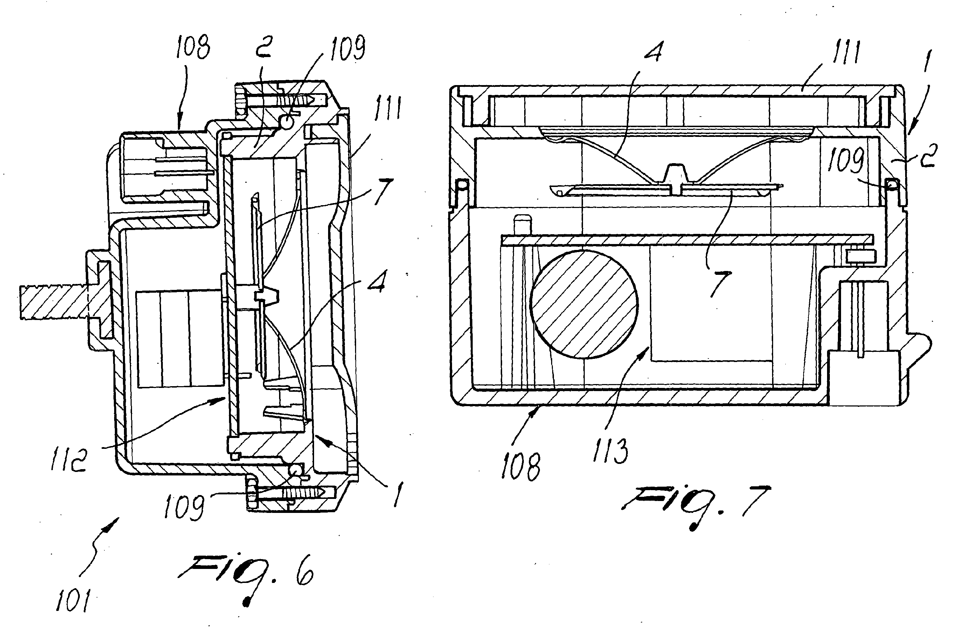

Figures 6 and 7 illustrate, only by way of example, two slightly different embodiments of the siren 101 that differ in the type and arrangement of the electronics, 112 and 113 respectively, associated with the emission device 1.

In practice it has been found that the sound emission device according to the invention fully achieves the intended aim and objects, since it allows to have a sound emission device that is absolutely impermeable, with reduced assembly costs and times by virtue of the characteristic of co-molding the membrane and the diffuser, and with frequency response optimization and repeatability.

The shape of the membrane, if it is changed, allows to also vary the passband thereof.

The device thus conceived is susceptible of numerous modifications and variations, within the scope of the appended claims. All the details may be replaced with other technically equivalent elements.

In practice, the materials used, as well as the contingent shapes and dimensions, may be any according to requirements and to the state of the art.

## Claims

1. A sound emission device for alarm systems for motor vehicles, motorcycles, enclosed spaces and the like, comprising a diffuser and at least one membrane accommodated inside said diffuser, said membrane being adapted to be made to vibrate by a transducer means, **characterized in that** said membrane and said diffuser are formed monolithically.

2. The device according to claim 1, **characterized in that** said membrane and said diffuser are co-molded in a single molding process without discontinuities.

3. The device according to claim 1, **characterized in that** said membrane is arranged so that the concavity is directed towards the inside of the diffuser.

4. The device according to claim 1, **characterized in that** said membrane is arranged so that the concavity is directed towards the outside of said diffuser.

5. The device according to one or more of the preceding claims, **characterized in that** said diffuser and said membrane are made of the same material.

6. The device according to one or more of the preceding claims, **characterized in that** said membrane and said diffuser are made of different materials.

7. The device according to one of the preceding claims, **characterized in that** said membrane and said diffuser are obtained from polymers for injection-molding.

8. A method for providing a sound emission device, particularly for anti-theft systems for motor vehicles, motorcycles, enclosed spaces and the like, **characterized in that** it comprises providing a diffuser body and a membrane by a co-molding process in order to obtain said diffuser and said membrane monolithically.

9. The method according to claim 8, **characterized in that** said co-molding process is a co-injection-molding process.

10. The method according to claim 8, **characterized in that** said diffuser and said membrane are made of the same material.

11. The method according to claim 8, **characterized in that** said diffuser and said membrane are made of different materials.

12. The method according to claim 8, **characterized in that** said diffuser and said membrane are made of a polymeric material that is suitable for injection-molding.

13. A siren, particularly for anti-theft systems for motor vehicles, motorcycles, enclosed spaces and the like, **characterized in that** it comprises a sound emission device comprising a diffuser and at least one membrane accommodated inside said diffuser, said membrane being adapted to be made to vibrate by a transducer means, said membrane and said diffuser being formed monolithically.

14. The siren according to claim 13, **characterized in that** said siren comprises a casing which is sealingly closed by said diffuser body.

15. The siren according to claim 14, **characterized in that** it comprises a seal member provided between said casing and said diffuser body.

16. The siren according to claim 15, **characterized in that** said seal member is an O-ring.

17. The siren according to claim 15, **characterized in that** it comprises a protective cover provided at the front of said diffuser body.

18. The siren according to one or more of claims 14-17, **characterized in that** said casing contains electronic means associated with said emission device.

19. An anti-theft system for motor vehicles, motorcycles, enclosed spaces and the like, **characterized in that** it comprises a sound emission device according to one or more of claims 1 to 7.

20. The use of a sound emission device according to one or more of claims 1 to 19 in an alarm system for motor vehicles, motorcycles, enclosed spaces and the like.
